# EUROPÄISCHE PATENTANMELDUNG

(11) **EP 3 617 343 A1**
(43) Veröffentlichungstag der Anmeldung: **04.03.2020**
(21) Anmeldenummer: 19190418.4
(22) Anmeldetag: 07.08.2019
(51) Int. Cl.: C23C 16/455, H01J 37/32, A61L 2/14

(54) **ANORDNUNG UND VERFAHREN ZUR BEHANDLUNG VON OBJEKTEN**

(30) Priorität: 21.08.2018 DE 102018120269
(71) Anmelder: Relyon Plasma GmbH, 93055 Regensburg (DE)
(72) Erfinder: NETTESHEIM, Stefan, 93051 Regensburg (DE)
(74) Vertreter: Reichert & Lindner Partnerschaft Patentanwälte

(57) **Zusammenfassung**

Es ist eine Anordnung (1) und ein Verfahren zur Behandlung von mindestens einem Objekt (8) offenbart. Hierzu ist eine lonisationskammer/Plasmakammer (4) vorgesehen, die über eine Hochspannungsleitung (3) mit der Hochspannungsquelle (2) verbunden ist. Eine erste Ventilgruppe (11) besitzt einen Knoten (16) und eine zweite Ventilgruppe (12) besitzt einen Knoten (17), wobei zwischen der ersten Ventilgruppe (11) und der zweiten Ventilgruppe (12) eine Pumpe (14) vorgesehen ist. Eine Behandlungskammer (6) ist fluide mit der ersten Ventilgruppe (11) und der zweiten Ventilgruppe (12) und die Ionisationskammer/Plasmakammer (4) ist ebenfalls fluide mit der ersten Ventilgruppe (11) und der zweiten Ventilgruppe (12) verbindbar.

## Beschreibung

Die vorliegende Erfindung betrifft eine Anordnung zur Behandlung von mindestens einem Objekt.

Ferner betrifft die Erfindung ein Verfahren zur Behandlung von mindestens einem Objekt.

Industriell werden Plasmaverfahren derzeit u. a. in der Medizintechnik, der Werkstoffherstellung und der Beleuchtungstechnik eingesetzt. Die Anwendung von Plasma erlaubt prinzipiell eine Reduktion mikrobieller Kontaminanten bei geringen Temperaturen, wobei die Wirkung primär auf den Oberflächen erzielt wird. Erste Versuchsreihen im Labormaßstab zur Plasmaanwendung im Lebensmittelbereich untersuchen hauptsächlich Möglichkeiten zur Inaktivierung unerwünschter Mikroorganismen bei hitzeempfindlichen Lebensmitteln, da herkömmliche thermische Dekontaminationsverfahren bei Produkten, wie beispielsweise frischem Obst und Gemüse, Fleisch oder Eiern, nicht oder nur begrenzt einsetzbar sind. Die Plasmaanwendung gilt auch als potenzielle Alternative zu anderen chemischen (z. B. Chloreinsatz) oder physikalischen Verfahren (z. B. Hochdruck-, Hochspannungsimpulse, ionisierende Bestrahlung). Als Vorteile des Plasmaverfahrens gelten u. a. eine hohe Wirksamkeit bei niedrigen Temperaturen (i. d. R. < 70 °C), gezielte und verbrauchsgerechte Bereitstellung, geringe Beeinflussung der inneren Produktmatrix sowie wasser- bzw. lösemittel- und rückstandsfreie sowie ressourceneffiziente Anwendung. Andere Verfahren, wie Hochdruck und/oder ionisierende Strahlung sind komplex oder kostspielig. Die Dekontamination mit UV-Licht ist oft nicht wirksam und durch Schatteneffekte eingeschränkt.

Die internationale Patentanmeldung WO 1995/009256 A1 befasst sich mit der Behandlung, insbesondere der Reinigung von Oberflächen, insbesondere Folien- und Metalloberflächen. Die Reinigung wird dadurch erreicht, dass eine dielektrisch behinderte Entladung auf die Oberfläche des Folienbandes einwirkt. Durch Einwirkung hochenergetischer geladener Teilchen und durch Einwirkung von Photonen aus dem UV-Spektralbereich wird z. B. der Öl- bzw. Fettfilm bereits nach kurzer Zeit so weit entfernt, dass eine Weiterverarbeitung möglich ist.

In dem europäischen Patent EP 1 337 281 B1 wird eine Verstärkung der reinigenden Wirkung von Plasma beschrieben. Die Verstärkung der indirekten Wirkung auf die zu reinigende oder zu sterilisierende Oberfläche wird durch Zumischen von additiven Gaskomponenten, wie z. B. Sauerstoff, Wasserdampf oder auch Edelgasen erreicht.

Die deutsche Offenlegungsschrift DE 100 36 550 A1 betrifft ein Sterilisationsverfahren bei dem die zu behandelnde Oberfläche einer Gasentladung ausgesetzt wird. Die Sterilisation wird in einer Wasserstoff- und sauerstoffhaltigen Gasatmosphäre bei einem bestimmten Druck durchgeführt. Ein Wirkungsoptimum wird insbesondere durch angefeuchtete Luft (N₂+ O₂ +H₂O) erreicht.

Die US-Patentanmeldung US 2015/0038584 A1 offenbart eine Vorrichtung zur Plasmabehandlung von Oberflächen von Objekten. Es ist dabei eine räumliche und zeitliche Trennung des Plasmaprozesses und der Exposition des zu reinigenden/sterilisierenden Objektes beschrieben.

Die US-Patentanmeldung US 2016/0220714 A1 offenbart eine Desinfektionsvorrichtung zur Plasmadesinfektion von Oberflächen mit einem Plasmagenerator. Zur Erzeugung eines desinfizierenden Plasmagasstroms steht der Plasmagasstrom mit dem Plasmagenerator in kommunizierender Verbindung. Es ist mindestens ein teilweise geschlossener Desinfektionsbereich vorgesehen, der zur Aufnahme der zu desinfizierenden Oberfläche ausgebildet ist. Die Desinfektionsvorrichtung besitzt einen Aerosolgenerator zur Erzeugung eines wässrigen Partikels enthaltenen Aerosolstroms. Der Aerosolgenerator steht mit dem Plasmagenerator in kommunizierender Verbindung, um den mit dem Aerosolstrom vermischten Plasmagasstrom im Desinfektionsbereich auf die zu desinfizierende Oberfläche zu leiten.

Zu diesem aktivierten Wasser können je nach Prozess für die Sterilisation noch zusätzliche Additive, wie z. B. Peressigsäure oder Wasserstoffperoxid, hinzugesetzt werden.

Die US-Patentanmeldung US 2003/0133832 A1 offenbart die Verwendung von freien Hydroxylradikalen zur Sterilisation bzw. Dekontamination. Die Hydroxylradikale weisen ein besonders hohes Oxidationspotential auf. Die Hydroxylradikale werden durch die photolytische Reaktion von Ozon mit Wasser unter UV-Licht gebildet.

Die deutsche Patentanmeldung DE 10 2008 037 898 A1 betrifft ein Verfahren sowie eine Vorrichtung zur Desinfektion oder Sterilisation von Verpackungsmaterial und/oder Behältern und/oder Filtermaterial, wobei das Material bzw. der Behälter mit einem in einem Plasmareaktor erzeugten Gas behandelt wird.

Die deutsche Patentanmeldung DE 10 2015 119 369 A1 betrifft eine Vorrichtung sowie ein System und ein Verfahren zur Behandlung eines Gegenstandes, insbesondere eines oder mehrerer Freiformkörper, mit Plasma. Die Vorrichtung dient zur Behandlung eines Gegenstandes mit Plasma und umfasst eine Hülleinrichtung, mit der ein im Wesentlichen gasdichter Aufnahmeraum ausgebildet oder ausbildbar ist, in den ein zu behandelnder Gegenstand aufgenommen werden kann. Des Weiteren umfasst die Vorrichtung eine erste Elektrode sowie eine zweite Elektrode, wobei die beiden Elektroden in Bezug zur Hülleinrichtung derart angeordnet sind, dass bei Anlage einer elektrischen Potenzialdifferenz an den Elektroden ein Plasma im Aufnahmeraum der Hülleinrichtung erzeugbar ist.

Die US-Patentanmeldung US 2017/112157 A1 offenbart ein Verfahren zur Behandlung einer Oberfläche mit einem reaktiven Gas. Das reaktive Gas wird aus kaltem Plasma bei Hochspannung aus einem Arbeitsgas hergestellt (HVCP).

Die deutsche Patentanmeldung DE 10 2014 213 799 A1 offenbart ein Haushaltskältegerät mit einer Lebensmittel-Behandlungseinheit sowie ein Verfahren zum Betreiben eines derartigen Haushaltskältegeräts. Das Haushaltskältegerät ist mit einem Innenraum zur Aufnahme von Lebensmitteln versehen, der durch Wände eines Innenbehälters begrenzt ist. Ferner ist eine Lebensmittel-Behandlungseinheit vorgesehen, wobei die Lebensmittel-Behandlungseinheit zur Einwirkung auf eine Oberfläche, von in dem Lagerbereich eingebrachten Lebensmitteln, in dem Haushaltskältegerät angeordnet ist, und so ausgebildet ist, dass das Einwirken eine Dekontamination von Pestiziden und/oder Schwermetallen im Lebensmittel ist.

Die deutsche Patentanmeldung DE 10 2005 061 247 A1 offenbart ein Verfahren und eine Vorrichtung zum Entkeimen von Lebensmitteln. Das Lebensmittel wird mit mindestens einem atmosphärischen Plasmastrahl beaufschlagt. Durch die im Plasmastrahl enthaltene Energie wird die Entkeimung der Oberfläche des Lebensmittels durchgeführt.

Die deutsche Patentanmeldung DE 11 2015 006 315 T5 offenbart eine Ladungsteilchenstrahleinrichtung mit einer Probenkammer und einer Ladungsteilchenkanonenkammer. Die Probenkammer wird in einen Hochvakuumzustand versetzt und über einen Haupteinlass mit einer Turbomolekularpumpe evakuiert. Dadurch soll es möglich sein, die Kontamination des Einrichtungsinnenraums sowohl beim Hochvakuums- als auch beim Grobvakuumszustand zu minimieren, wodurch es möglich ist, dass eine Kontamination der betrachteten Probe verhindert wird.

Die US-Patentanmeldung US 2004/0084148 A1 offenbart eine Vorrichtung, die mit Niederdruckplasma arbeitet. Ein Substrat in Form eines Films wird von außen in die Kammer eingebracht. Während Reaktionsgas eingebracht wird, wird die Kammer evakuiert. Mittels eine Hochfrequenz kann das Plasma erzeugt werden.

Die deutsche Übersetzung DE 60 2005 003 223 T2 des europäischen Patents EP 1 729 894 B2 offenbart die Abscheidung eines Polymerisats auf einem Substrat. Das Monomer wird in eine Plasmaabscheidungskammer eingeführt. In der Kammer wird eine Glimmentladung durch Anlegen einer Spannung in Form eines gepulsten Feldes gezündet. Die Polymerschicht bildet sich an der Oberfläche des Substrats aus.

Die deutsche Patentanmeldung DE 43 18 086 A1 offenbart die Herstellung einer polymeren Deckschicht auf der inneren Oberfläche eines Kunststoffhohlkörpers durch Niederdruck-Plasmapolymerisation. Ein Plasma wird durch Mikrowellen erzeugt und bildet unter den jeweiligen Plasmabedingungen polymerisierbare, im Wesentlichen unpolare gas- und/oder dampfförmige Ausgangssubstanz. Dabei kann die schichtbildende Gasatmosphäre zwei oder mehr Komponenten enthalten, die einerseits überwiegend zu Kettenwachstum neigen und andererseits dazu neigen, Verzweigungs- bzw. Vernetzungsstellen zu bilden.

Die US-Patentanmeldung US 2004/0129212 A1 offenbart eine Vorrichtung mit der ein chemisch reaktiver Vorgänger an eine zu behandelnde Oberfläche geliefert werden kann. Es sind besonders gestaltete Auslässe vorgesehen, damit sich der Vorgänger gleichmäßig auf der Oberfläche niederschlägt.

Das US-Patent US 6,083,363 B1 zeigt eine Vorrichtung mit der eine gleichmäßige und einheitliche Plasmabehandlung einer Oberfläche möglich ist. Die Behandlung der Oberfläche soll schonend sein, hierzu ist zwischen der Plasmakammer und der Reaktionskammer ein Mittel vorgesehen, mit dem Ionen extrahiert werden können.

Die US-Patentanmeldung US 2012/0270406 A1 offenbart ein Verfahren zum Reinigen eines Plasmabehandlungsapparates.

Alle nicht thermischen Verfahren (chemisch, plasmachemisch, physikalisch oder optisch) sind umso wirkungsvoller, je höher die Konzentration der wirksamen Spezies ist und je höher die Expositionszeit der kontaminierten Oberfläche ist. Dabei werden bei mikrobiologischen Organismen oft Schwellwerte beobachtet, unterhalb derer auch bei längerer Exposition die Wirkung verschwindet. Eine vollkommene Dekontamination kann dadurch nicht erreicht werden.

Bei einer direkten Behandlung mit chemischen Gasmischungen oder Flüssigkeiten, wie z. B. Etylenoxid oder H₂O₂ kann die Konzentration in einem weiten Bereich frei an den Prozess angepasst werden. Allerdings müssen dann gegebenenfalls gesundheitsgefährdende Konzentrationen sicher gehandhabt werden.

Bei UV- oder Entladungsverfahren ist die Konzentration der erzeugten Spezies stark abhängig von der Leistung und der Leistungsdichte, die nicht beliebig zu steigern ist.

Der Erfindung liegt die Aufgabe zugrunde, eine Anordnung zur Behandlung mindestens eines Objekts zu schaffen, mittels dem alle Oberflächenbereiche des Objekts bei der Behandlung ausgesetzt werden können.

Die obige Aufgabe wird durch eine Anordnung zur Behandlung von mindestens einem Objekt gelöst, die die Merkmale des Anspruchs 1 umfasst.

Eine weitere Aufgabe der Erfindung ist, ein Verfahren zur Behandlung mindestens eines Objekts zu schaffen, wobei während der Behandlung des Objekts sämtliche Oberflächenbereiche des Objekts der Behandlung ausgesetzt werden.

Diese Aufgabe wird durch ein Verfahren zur Behandlung von Objekten gelöst, das die Merkmale des Anspruchs 6 umfasst.

Eine mögliche Ausführungsform der Anordnung zur Behandlung von mindestens einem Objekt umfasst eine lonisationskammer/Plasmakammer, die über eine Hochspannungsleitung mit der Hochspannungsquelle verbunden ist. In der lonisationskammer/Plasmakammer ist eine Entladungsstruktur vorgesehen, an der die Hochspannung anliegt. Mit der Entladungsstruktur kann das Prozessgas in der lonisationskammer/Plasmakammer ionisiert oder mit dem Prozessgas ein Plasma erzeugt werden. Die Ausgestaltung der Entladungsstruktur ist dabei hinlänglich aus dem Stand der Technik bekannt und braucht hier nicht mehr im Detail beschrieben werden. Natürlich kann auch statt einer konventionell aufgebauten Entladungsstruktur eine direkte piezoelektrische Entladung eingesetzt werden, die ebenfalls im Stand der Technik hinreichend beschrieben worden ist.

Ferner umfasst die Anordnung eine erste Ventilgruppe mit mehreren Ventilen und eine zweite Ventilgruppe mit mehreren Ventilen, wobei die erste Ventilgruppe und die zweite Ventilgruppe jeweils einen Knoten definieren. Zwischen dem Knoten der ersten Ventilgruppe und dem Knoten der zweiten Ventilgruppe ist eine Pumpe vorgesehen. Die Pumpe kann zur Unterstützung des Gastransports innerhalb der Anordnung oder der Erzeugung von Unterdruck bei Elementen der Anordnung verwendet werden. Eine Behandlungskammer ist fluide über ein Ventil der ersten Ventilgruppe und ein Ventil der zweiten Ventilgruppe verbunden. Ebenso ist die lonisationskammer/Plasmakammer fluide über ein Ventil der ersten Ventilgruppe und ein Ventil der zweiten Ventilgruppe verbunden. Über die erste und die zweite Ventilgruppe kann z. B. der lonisationskammer/Plasmakammer oder der Behandlungskammer gezielt und gesteuert Gas zugeführt oder abgeführt werden. Die Behandlungskammer ist mit einer Schleuse zum Einbringen eines Objekts in die Behandlungskammer versehen.

Der Vorteil der erfindungsgemäßen Anordnung ist, dass bei der Behandlungskammer ein Unterdruck vorherrscht und diese dann aktiv, mittels des Unterdrucks, mit dem aktivierten Prozessgas befüllt werden kann. Dadurch ist es möglich, dass der Raum in der Behandlungskammer komplett gefüllt und somit alle Spalten oder Vertiefungen am Objekt erreicht werden können. Hinzu kommt, dass die Anordnung ein geschlossenes System darstellt. Weiterhin ist die freie Wahl des Prozessdruckes in der lonisationskammer/Plasmakammer während der Ionisation, die bei abgesenktem Druck begünstigt ist, von Vorteil. Natürlich kann das Objekt auch während der Behandlung mit dem aktivierten Prozessgas bewegt werden um den Kontakt mit allen Oberflächen des Objektes zu begünstigen.

Von Vorteil bei der erfindungsgemäßen Anordnung ist auch, dass die Partialdrücke und damit die absoluten Mengen bestimmter stofflicher Komponenten, die für die Behandlung der Objekte beigemischt werden, während des Prozesses genau bestimmt und dosiert werden können.

Ein Gaseinlass, über den Prozessgas zur Verfügung gestellt wird, ist über ein Ventil der ersten Ventilgruppe verbunden. Ein Gasauslass, über den z. B. überschüssiges Prozessgas oder Gas aus der Behandlungskammer abgeleitet wird, ist über ein Ventil mit der zweiten Ventilgruppe verbunden. Der Vorteil des Gaseinlasses ist, dass hier ein Gasgemisch, wie z. B. angefeuchtete Luft erzeugt werden kann. Darüber hinaus kann das Eintrittsgas (Prozessgas) gefiltert werden. Im Gasauslass kann ein Reststofffilter oder Schmutzfilter integriert sein.

Die erste Ventilgruppe umfasst mehrere Ventile. Ebenso umfasst die zweite Ventilgruppe mehrere Ventile. Die Ventile der ersten und zweiten Ventilgruppe können, z. B. von einer Steuerung, bedarfs- und zeitgerecht gesteuert werden, um den Gasstrom zwischen der lonisationskammer/Plasmakammer, der Behandlungskammer, dem Gaseinlass und/oder dem Gasauslass bedarfs- und zeitgerecht zu regeln. Die Steuerung kann z. B. aus einen Computer, Tablet oder Mobiltelefon realisiert werden. Die Ventile sind mit Aktoren versehen, damit die geforderte Ventilstellung eingestellt werden kann. Ebenso kann die Steuerung mit der Hochspannungsquelle, der Entladungsstruktur, der lonisationskammer/Plasmakammer oder der Behandlungskammer kommunikativ verbunden sein. Die kommunikative Verbindung kann drahtgebunden oder drahtlos sein.

Die Behandlungskammer ist über eine Leitung fluide mit einem Ventil der ersten Ventilgruppe und über eine Leitung fluide mit einem Ventil der zweiten Ventilgruppe verbunden.

Die lonisationskammer/Plasmakammer ist über eine Leitung fluide mit einem Ventil der ersten Ventilgruppe und über eine Leitung fluide mit einem Ventil der zweiten Ventilgruppe verbunden.

Der Gaseinlass ist über eine Leitung fluide mit einem Ventil der ersten Ventilgruppe verbunden. Der Gasauslass ist über eine Leitung fluide mit einem Ventil der zweiten Ventilgruppe verbunden.

Das erfindungsgemäße Verfahren zur Behandlung von mindestens einem Objekt, zeichnet sich dadurch aus, dass zunächst mindestens ein Objekt in eine Behandlungskammer verbracht wird. Die Behandlungskammer kann hierzu eine Schleuse aufweisen. Nachdem das Objekt in der Behandlungskammer ist, kann mit einer Pumpe ein Unterdruck in der Behandlungskammer erzeugt werden. Eine lonisationskammer/Plasmakammer wird über einen Gaseinlass mit einem Prozessgas befüllt. Mit einer Entladungsstruktur wird in der lonisationskammer/Plasmakammer das Prozessgas aktiviert, so dass eine Ionisation oder Aktivierung des Prozessgases erfolgt. Das aktivierte bzw. ionisierte Prozessgas wird von der lonisationskammer/Plasmakammer in die Behandlungskammer gesaugt. Das Saugen des aktivierten Prozessgases erfolgt durch den in der Behandlungskammer herrschenden Unterdruck. Nach einer Einwirkzeit des aktivierten bzw. ionisierten Prozessgases auf das Objekt, wird das Prozessgas aus der Behandlungskammer abgepumpt. Anschließend wird die Behandlungskammer belüftet, und das mindestens eine behandelte Objekt wird aus der Behandlungskammer entnommen.

Die Pumpe ist zwischen einem Knoten der ersten Ventilgruppe und einem Knoten der zweiten Ventilgruppe vorgesehen. Durch die Pumpe kann zumindest wahlweise an die Behandlungskammer ein Unterdruck angelegt werden. Auch kann nach einer Einwirkzeit auf das mindestens eine Objekt, das aktivierte bzw. ionisierte Prozessgas, aus der Behandlungskammer abgepumpt werden.

Mittels der Pumpe kann ein Anreicherungszyklus (Nachlieferung von Prozessgas in die lonisationskammer/Plasmakammer) von aktiviertem bzw. ionisiertem Prozessgas in der lonisationskammer/Plasmakammer durchgeführt werden. Beim Anreicherungszyklus ist die Entladungsstruktur in der lonisationskammer/Plasmakammer aktiviert. Hierzu sind ein Ventil der ersten Ventilgruppe und ein Ventil der zweiten Ventilgruppe geöffnet, so dass die lonisationskammer/Plasmakammer mit der Pumpe über einen Kreislauf fluide verbunden wird.

Das mindestens eine Objekt wird über eine Schleuse in die Behandlungskammer eingebracht. Hierzu sind ein Ventil der ersten Ventilgruppe und ein Ventil der zweiten Ventilgruppe geöffnet, so dass die Behandlungskammer eine fluide Verbindung über die zweite Ventilgruppe mit dem Gasauslass ausbildet.

Für das Erzeugen des Unterdrucks in der Behandlungskammer wird die Pumpe aktiviert. Dabei sind ein Ventil der ersten Ventilgruppe und ein Ventil der zweiten Ventilgruppe geöffnet, so dass die Behandlungskammer eine fluide Verbindung zum Gasauslass ausbildet.

Zum Befüllen der lonisationskammer/Plasmakammer mit dem Prozessgas sind ein Ventil der ersten Ventilgruppe und ein Ventil der zweiten Ventilgruppe geöffnet. Dadurch ist ein Gaseinlass mittels einer fluiden Verbindung mit der lonisationskammer/Plasmakammer verbunden.

Bei der aktivierten Entladungsstruktur in der lonisationskammer/Plasmakammer erfolgt eine Ionisation oder Aktivierung des Prozessgases. Hierzu sind ein Ventil der ersten Ventilgruppe und ein Ventil der zweiten Ventilgruppe geöffnet. Dadurch besteht eine fluide Verbindung von der Behandlungskammer über die Pumpe zum Gasauslass.

In einem nächsten Schritt wird das aktivierte bzw. ionisierte Prozessgas aus der lonisationskammer/Plasmakammer in die Behandlungskammer gesaugt. Das Saugen kann deshalb erfolgen, da in der Behandlungskammer ein Unterdruck vorliegt. Hierzu sind ein Ventil und ein weiteres Ventil der ersten Ventilgruppe geöffnet, so dass eine fluide Verbindung von der lonisationskammer/Plasmakammer zu der Behandlungskammer besteht. Das Ventil und das weitere Ventil der ersten Ventilgruppe bleiben, während einer Einwirkzeit des ionisierten bzw. aktivierten Prozessgases auf das Objekt, in der Behandlungskammer geöffnet.

Zum Abpumpen der Behandlungskammer sind ein Ventil der ersten Ventilgruppe und ein Ventil der zweiten Ventilgruppe geöffnet. Dadurch wird eine fluide Verbindung von der Behandlungskammer über die Pumpe zu einem Gasauslass gebildet.

Zum Belüften der Behandlungskammer sind ein Ventil und ein weiteres Ventil der zweiten Ventilgruppe geöffnet. Dadurch wird eine fluide Verbindung von der Behandlungskammer zu einem Gasauslass gebildet.

Es ist bei geeigneter Wahl des Prozesses möglich, alle Prozesse bei Unterdruck, bezogen auf die Umgebung oder bei Überdruck, bezogen auf die Umgebung, ablaufen zu lassen.

Eine Steuerung kann kommunikativ mit der Anordnung verbunden werden. Mit der Steuerung können zumindest die Entladungsstruktur der lonisationskammer/Plasmakammer, die Ventile der ersten Ventilgruppe und die Ventile der zweiten Ventilgruppe zeit- und bedarfsgerecht gesteuert werden.

Im Folgenden sollen Ausführungsbeispiele die Erfindung und ihre Vorteile anhand der beigefügten Figuren näher erläutern. Die Größenverhältnisse in den Figuren entsprechen nicht immer den realen Größenverhältnissen, da einige Formen vereinfacht und andere Formen zur besseren Veranschaulichung vergrößert im Verhältnis zu anderen Elementen dargestellt sind. Dabei zeigen:
- **Figur 1**: eine schematische Darstellung einer möglichen Ausführungsform der erfindungsgemäßen Anordnung zur Behandlung von mindestens einem Objekt;
- **Figur 2**: eine schematische Darstellung der Zuordnung einer Steuerung zu der erfindungsgemäßen Anordnung;
- **Figur 3**: ein Ablaufdiagramm des erfindungsgemäßen Verfahrens zur Behandlung von mindestens einem Objekt;
- **Figur 4**: eine Darstellung der Ausbildung eines Kreislaufs mit der lonisationskammer/Plasmakammer;
- **Figur 5**: eine Darstellung der fluiden Verbindung der Behandlungskammer mit dem Gasauslass;
- **Figur 6**: eine Darstellung der fluiden Verbindung zum Erzeugen des Unterdrucks in der Behandlungskammer;
- **Figur 7**: eine Darstellung der fluiden Verbindung des Gaseinlasses mit der lonisationskammer/Plasmakammer
- **Figur 8**: eine Darstellung der fluiden Verbindung der Behandlungskammer zum Gasauslass;
- **Figur 9**: eine Darstellung der fluiden Verbindung von der lonisationskammer/Plasmakammer zu der Behandlungskammer;
- **Figur 10**: eine Darstellung der fluiden Verbindung zum Abpumpen des Prozessgases aus der Behandlungskammer; und
- **Figur 11**: eine Darstellung der fluiden Verbindung zum Belüften der Behandlungskammer.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figuren erforderlich sind. In der nachstehenden Figurenbeschreibung werden die Anordnung und das Verfahren mit einer ersten Ventilgruppe beschrieben, die drei Ventile umfasst, und einer zweiten Ventilgruppe, die ebenfalls drei Ventile umfasst. Dies dient lediglich der Beschreibung und soll nicht als Beschränkung der Erfindung aufgefasst werden.

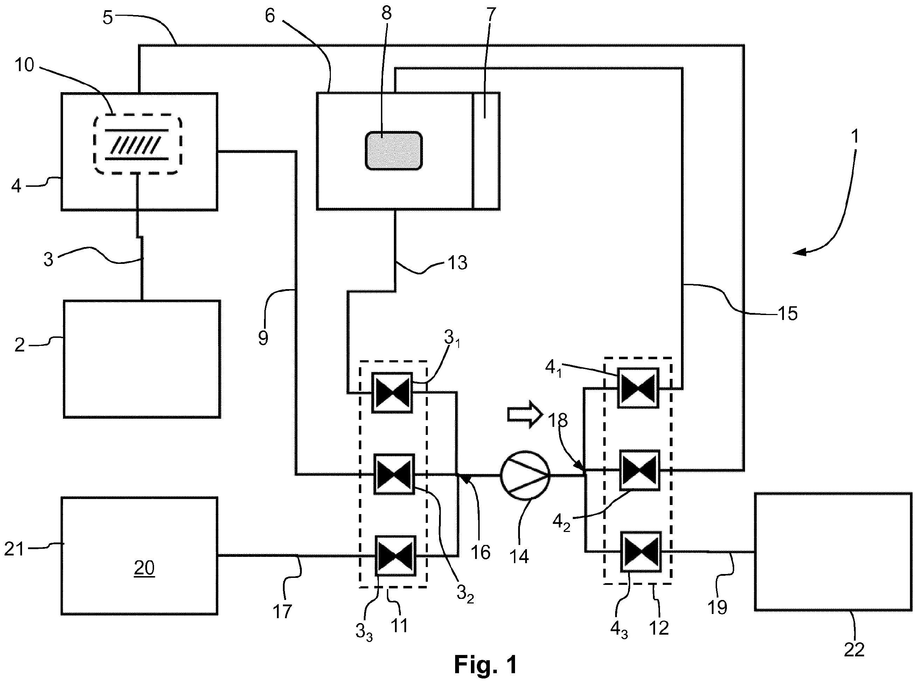

**Figur 1** zeigt eine schematische Darstellung einer möglichen Ausführungsform der erfindungsgemäßen Anordnung 1 zur Behandlung von mindestens einem Objekt 8. Die Anordnung 1 besitzt eine Ionisationskammer/Plasmakammer 4, in der eine Entladungsstruktur 10 vorgesehen ist. Eine Hochspannungsquelle 2 ist über eine Hochspannungsleitung 3 mit der Entladungsstruktur 10 in der lonisationskammer/Plasmakammer 4 verbunden. Die Anordnung 1 umfasst ferner eine Behandlungskammer 6, die mit einer Schleuse 7 versehen ist. Über die Schleuse 7 kann mindestens ein Objekt 8 in die Behandlungskammer 6 verbracht werden. Eine erste Ventilgruppe 11 und eine zweiten Ventilgruppe 12 ist der Anordnung 1 zugeordnet. Dabei hat die erste Ventilgruppe 11 einen Knoten 16 und die zweite Ventilgruppe 12 einen Knoten 18 ausgebildet. Der erste Knoten 16 und der zweite Knoten 18 definieren sich durch die Zusammenführung der Leitungen von den Ventilen 3₁, 3₂ und 3₃ der ersten Ventilgruppe 11 bzw. durch die Zusammenführung der Leitungen von den Ventilen 4₁, 4₂ und 4₃ der zweiten Ventilgruppe 12. Eine Pumpe 14 verbindet über die Knoten 16 und 18 die erste Ventilgruppe 11 mit der zweiten Ventilgruppe 12.

In der Anordnung 1 ist die Behandlungskammer 6 fluide mit der ersten Ventilgruppe 11 und der zweiten Ventilgruppe 12 verbunden, Die lonisationskammer/Plasmakammer 4 ist ebenfalls fluide mit der ersten Ventilgruppe 11 und der zweiten Ventilgruppe 12 verbunden. Ferner sind ein Gaseinlass 21 mit der ersten Ventilgruppe 11 und ein Gasauslass 22 mit der zweiten Ventilgruppe 12 verbunden.

Die Behandlungskammer 6 ist über eine Leitung 13 fluide mit dem ersten Ventil 3₁ der ersten Ventilgruppe 11 und über eine Leitung 15 fluide mit dem ersten Ventil 4₁ der zweiten Ventilgruppe 12 verbunden. Die Ionisationskammer/Piasmakammer 4 ist über eine Leitung 9 fluide mit dem zweiten Ventil 3₂ der ersten Ventilgruppe 11 und über eine Leitung 5 fluide mit dem zweiten Ventil 4₂ der zweiten Ventilgruppe 12 verbunden. Der Gaseinlass 21 ist über eine Leitung 17 fluide mit dem dritten Ventil 3₃ der ersten Ventilgruppe 11 verbunden. Der Gasauslass 22 ist über eine Leitung 19 fluide mit dem dritten Ventil 4₃ der zweiten Ventilgruppe 12 verbunden.

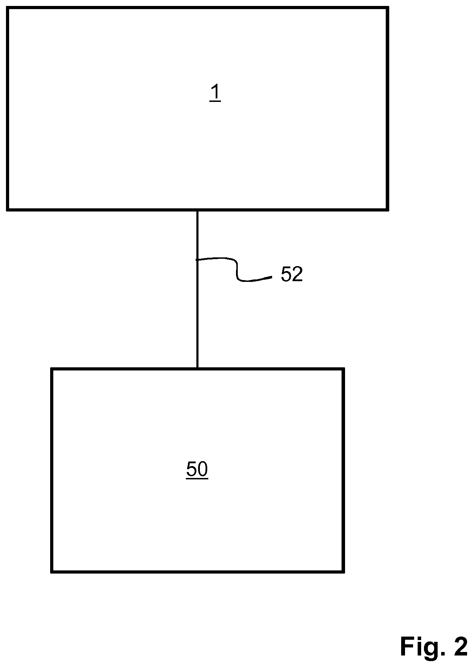

**Figur 2** zeigt eine schematische Darstellung der Zuordnung einer Steuerung 50 zu der erfindungsgemäßen Anordnung 1. Über eine Kommunikationsverbindung 52 ist die Steuerung 50 mit der Anordnung 1 verbunden. Mit der Steuerung 50 können zumindest die Ventile 3₁, 3₂ und 3₃ der ersten Ventilgruppe 11, die Ventile 4₁, 4₂ und 4₃ der zweiten Ventilgruppe 12, die Hochspannungsquelle 2, die mit der Entladungsstruktur 10 und die Pumpe 14 bedarfs- und zeitgerecht gesteuert und geregelt werden. Die Kommunikationsverbindung 52 kann drahtgebunden oder drahtlos ausgebildet sein.

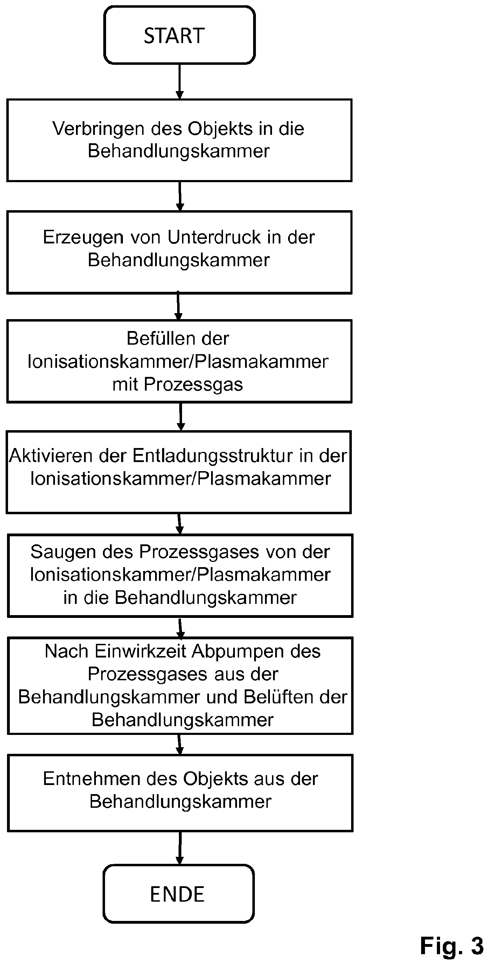

In **Figur 3** ist ein Ablaufdiagramm des erfindungsgemäßen Verfahrens zur Behandlung von mindestens einem Objekt 8 dargestellt. Zunächst wird das mindestens eine Objekt 8 in eine Behandlungskammer 6 der Anordnung 1 zur Behandlung verbracht. Dies erfolgt über eine Schleuse 7. Nachdem das mindestens eine Objekt 8 sind in der Behandlungskammer 6 befindet und die Schleuse 7 geschlossen ist, wird eine Pumpe 14 aktiviert. Mit der Pumpe 14 wird ein Unterdruck in der Behandlungskammer 6 erzeugt. Zum Erzeugen des Unterdrucks werden das erste Ventil 3₁ der ersten Ventilgruppe 11 und das dritte Ventil 4₃ der zweiten Ventilgruppe 12 geöffnet, so dass die Behandlungskammer 6 eine fluide Verbindung 103 zum Gasauslass 22 hin ausbildet. Die übrigen Ventile 3₂ und 3₃ der ersten Ventilgruppe 11 und die Ventile 4₁ und 4₂ der zweiten Ventilgruppe 12 bleiben geschlossen.

Dann wird die lonisationskammer/Plasmakammer 4 mit einem Prozessgas 20 über den Gaseinlass 21 befüllt. Hierzu sind ein drittes Ventil 3₃ der ersten Ventilgruppe 11 und ein zweites Ventil 4₂ der zweiten Ventilgruppe 12 geöffnet. Der Gaseinlass 21 ist somit über die ersten Ventilgruppe 11 und die zweite Ventilgruppe 12 fluide mit der lonisationskammer/Plasmakammer 4 verbunden. Die Entladungsstruktur 10 in der lonisationskammer/Plasmakammer 4 wird über die Hochspannungsquelle 2 aktiviert, so dass eine Ionisation oder Aktivierung des Prozessgases 20 erfolgt. Hier sind die übrigen Ventile 3₁ und 3₂ der ersten Ventilgruppe 11 und die Ventile 4₁ und 4₃ der zweiten Ventilgruppe 12 geschlossen.

Das aktivierte bzw. ionisierte Prozessgas 20 wird mittels Unterdruck von der lonisationskammer/Plasmakammer 4 in die Behandlungskammer 6 gesaugt. Der Unterdruck in der Behandlungskammer 6 sorgt dafür, dass das aktivierte bzw. ionisierte Prozessgas 20 von der lonisationskammer/Plasmakammer 4 in die Behandlungskammer 6 gelangt. Hierzu sind das erste Ventil 3₁ und das zweite Ventil 3₂ der ersten Ventilgruppe 11 geöffnet, somit sind die lonisationskammer/Plasmakammer 4 und die Behandlungskammer 6 miteinander fluide verbunden. Das erste Ventil 3₁ und das zweite Ventil 3₂ bleiben während einer Einwirkzeit geöffnet. Das Ventil 3₃ der ersten Ventilgruppe 11 und die Ventile 4₁, 4₂ und 4₃ der zweiten Ventilgruppe 12 bleiben geschlossen.

Nach der Einwirkzeit des aktivierten bzw. ionisierten Prozessgases 20 auf das Objekt 8 wird das Prozessgases 20 aus der Behandlungskammer 6 abgepumpt. Anschließend wird die Behandlungskammer 6 belüftet. Letztendlich wird das mindestens eine behandelte Objekt 8 über die Schleuse 7 der Behandlungskammer 6 entnommen.

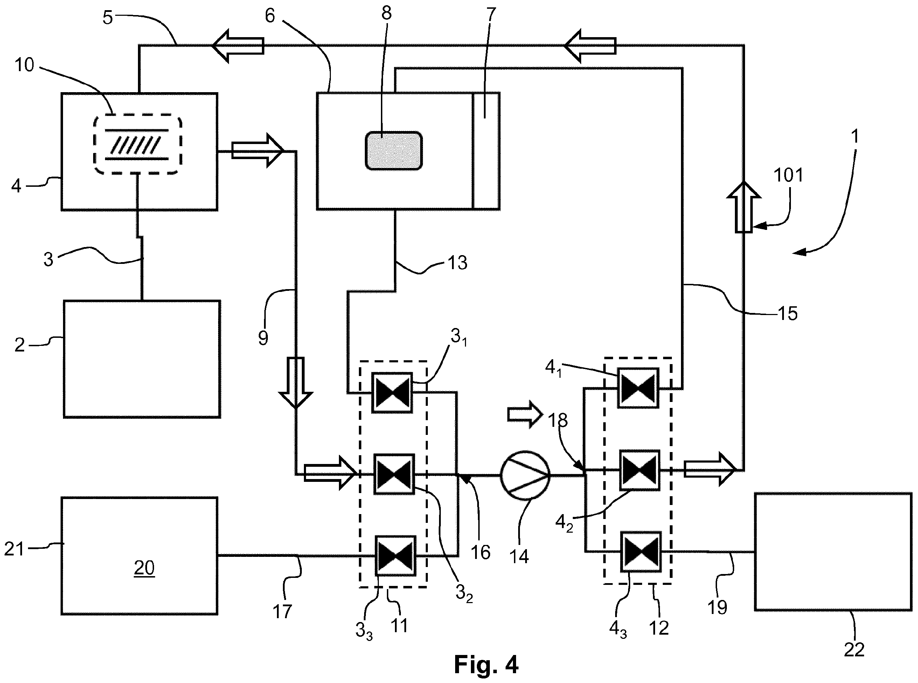

**Figur 4** zeigt eine Darstellung der Ausbildung eines Kreislaufs 101 in der Anordnung 1, wobei die lonisationskammer/Plasmakammer 4 in den Kreislauf 101 eingebunden ist. Mittels der Pumpe 14 wird ein Anreicherungszyklus von aktivierten bzw. ionisierten Prozessgas 20 in der Ionisationskammer/Plasmakammer 4 durchgeführt. Für den Anreicherungszyklus ist das zweite Ventil 3₂ der ersten Ventilgruppe 11 und das zweites Ventil 4₂ der zweiten Ventilgruppe 12 geöffnet. Dadurch ist der Kreislauf 101 definiert, so dass die lonisationskammer/Plasmakammer 4 und die Pumpe 14 fluide miteinander verbunden sind.

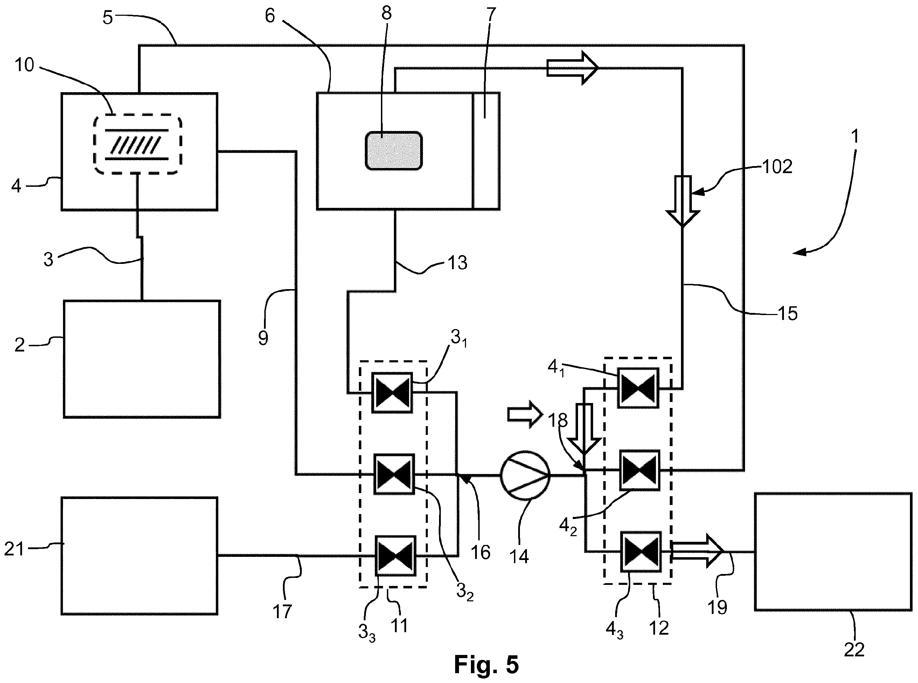

In **Figur 5** ist eine fluide Verbindung 102 der Behandlungskammer 6 mit dem Gasauslass 22 dargestellt. Zur Realisation der fluiden Verbindung 102 sind das erste Ventil 4₁ und das dritte Ventil 4₃ der zweiten Ventilgruppe 12 geöffnet, so dass die Behandlungskammer 6 die fluide Verbindung 102 über die zweite Ventilgruppe 12 mit dem Gasauslass 22 ausbildet.

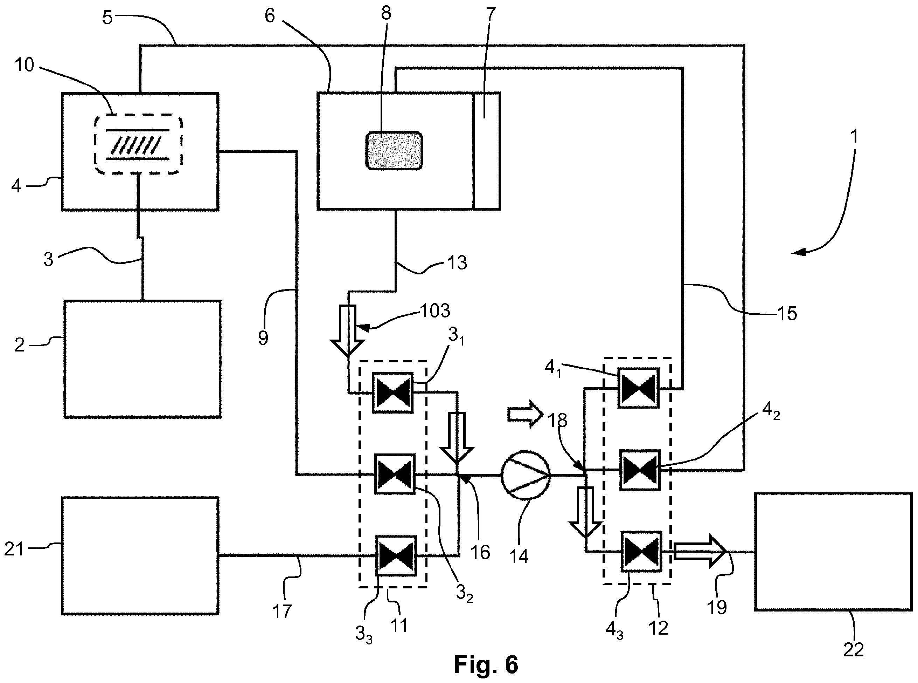

**Figur 6** zeigt eine Darstellung der fluiden Verbindung 103 zum Erzeugen des Unterdrucks in der Behandlungskammer 6. Die fluide Verbindung 103 läuft von der Behandlungskammer 6 über das erste Ventil 3₁ der ersten Ventilgruppe 11, die Pumpe 14 und das dritte Ventil 4₃ der zweiten Ventilgruppe 12 zum Gasauslass 22. Der Unterdruck in der Behandlungskammer 6 wird mittels der Pumpe 14 erzeugt. Dabei sind das erste Ventil 3₁ der ersten Ventilgruppe 11 und das dritte Ventil 4₃ der zweiten Ventilgruppe 12 geöffnet.

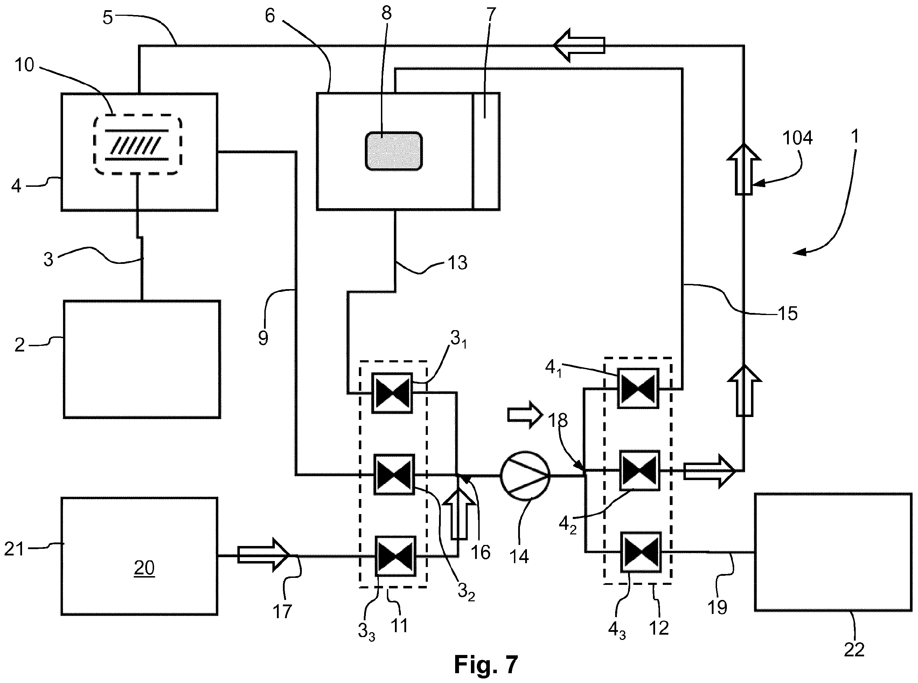

In **Figur 7** ist die fluide Verbindung 104 des Gaseinlasses 21 mit der lonisationskammer/Plasmakammer 4 dargestellt. Die fluide Verbindung 104 verläuft vom Gaseinlass 21 über das dritte Ventil 3₃ der ersten Ventilgruppe 11 und das zweite Ventil 4₂ der zweiten Ventilgruppe 12 zu der Ionisationskammer/Plasmakammer 4. Mittels der fluiden Verbindung 104 kann die Ionisationskammer/Plasmakammer 4 mit dem Prozessgas 20 befüllt werden. Das dritte Ventil 3₃ der ersten Ventilgruppe 11 und das zweite Ventil 4₂ der zweiten Ventilgruppe 12 sind geöffnet.

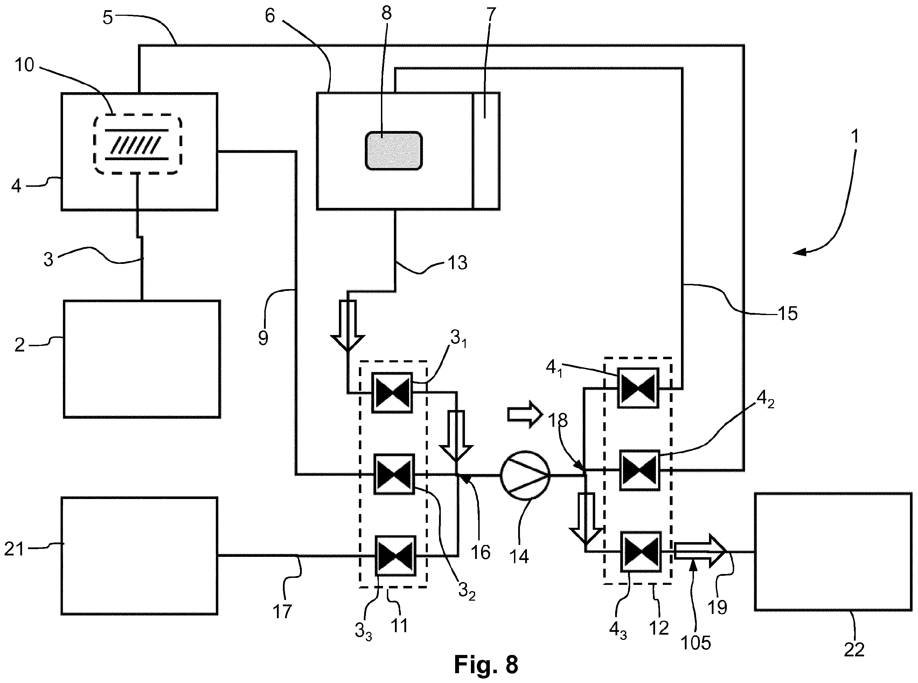

**Figur 8** ist eine Darstellung der fluiden Verbindung 105 der Behandlungskammer 6 zum Gasauslass 22. Bei der aktivierten Entladungsstruktur 10 in der lonisationskammer/Plasmakammer 4 erfolgt eine Ionisation oder Aktivierung des Prozessgases 20. Die fluiden Verbindung 105 verläuft von der Behandlungskammer 6 über eine Leitung 13 zum ersten Ventil 31 der ersten Ventilgruppe 11, der Pumpe 14 und das dritte Ventil 4₃ der zweiten Ventilgruppe 12 zum Gasauslass 22. Hierzu sind das erste Ventil 3₁ der ersten Ventilgruppe 11 und das dritte Ventil 4₃ der zweiten Ventilgruppe 12 geöffnet.

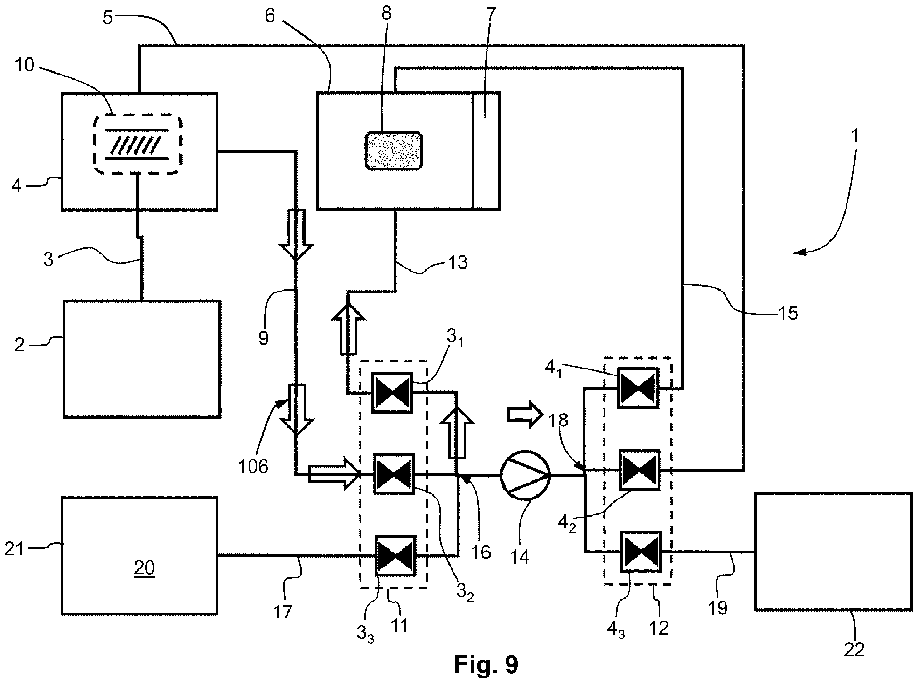

**Figur 9** ist eine Darstellung der fluiden Verbindung 106 von der lonisationskammer/Plasmakammer 4 zur Behandlungskammer 6. Das aktivierte bzw. ionisierte Prozessgas 20 wird aus der lonisationskammer/Plasmakammer 4 in die Behandlungskammer 6 gesaugt. Hierzu verläuft die fluide Verbindung 106 von der lonisationskammer/Plasmakammer 4 über das zweite Ventil 3₂ und das erste Ventil 3₁ der ersten Ventilgruppe 11 zur Behandlungskammer 6. Dabei sind das erste Ventil 31 und das zweite Ventil 3₂ der ersten Ventilgruppe 11 geöffnet. Das erste Ventil 3₁ und das zweite Ventil 3₂ bleiben während einer Einwirkzeit des aktivierten bzw. ionisierten Prozessgas 20 auf das Objekt in der Behandlungskammer 6 geöffnet.

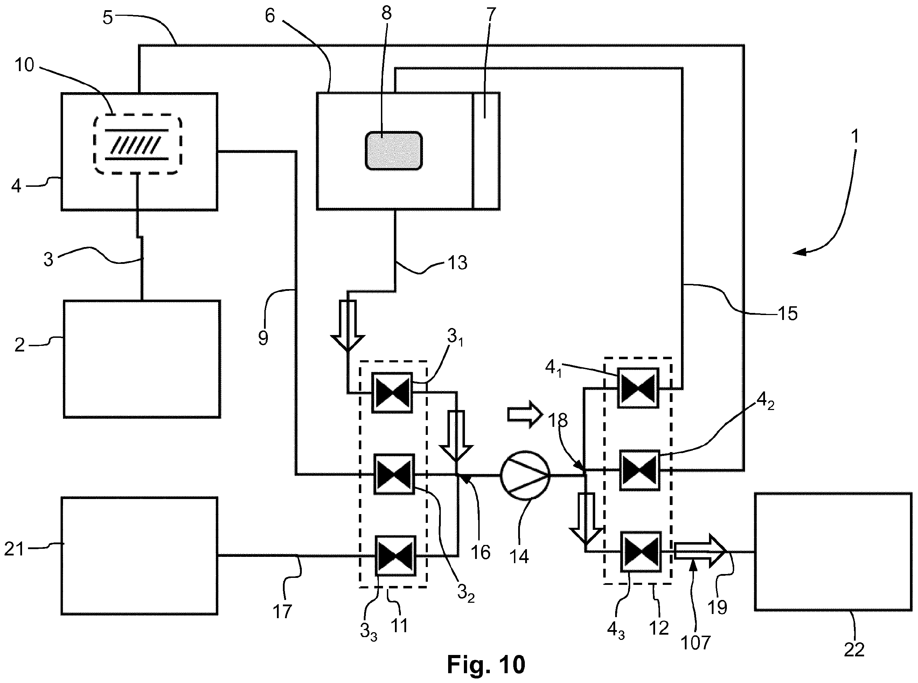

**Figur 10** ist eine Darstellung der fluiden Verbindung 107 zum Abpumpen des Prozessgases 20 aus der Behandlungskammer 6. Die fluide Verbindung 107 zum Abpumpen der Behandlungskammer 6 verläuft von der Behandlungskammer 6 über das erste Ventil 3₁ der ersten Ventilgruppe 11, die Pumpe 14 und das dritte Ventil 4₃ der zweiten Ventilgruppe 12 zum Gasauslass 22. Das erste Ventil 31 der ersten Ventilgruppe 11 und das dritte Ventil 4₃ der zweiten Ventilgruppe 12 sind geöffnet.

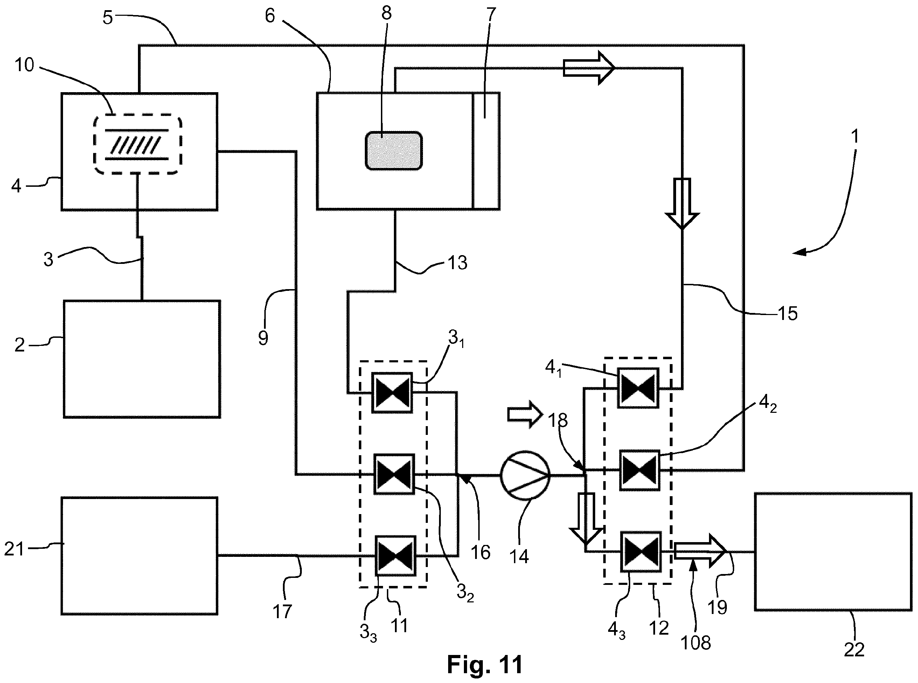

In **Figur 11** ist eine Darstellung der fluiden Verbindung 108 zum Belüften der Behandlungskammer 6 gezeigt. Die fluiden Verbindung 108 verläuft zum Belüften von der Behandlungskammer 6 über das erste Ventil 4₁ und das dritte Ventil 4₃ der zweiten Ventilgruppe 12 zum Gasauslass 22. Das erste Ventil 4₁ und das dritte Ventil 4₃ der zweiten Ventilgruppe 12 sind dabei geöffnet.

Die Erfindung wurde in Bezug auf bevorzugte Ausführungsformen beschrieben. Es ist für einen Fachmann jedoch selbstverständlich, dass Änderungen und Abwandlungen gemacht werden können, ohne dabei den Schutzbereich der nachstehenden Schutzansprüche zu verlassen.

### Bezugszeichenliste

- 1: Anordnung
- 2: Hochspannungsquelle
- 3: Hochspannungsleitung
- 3₁. 3₂,...,3_{N}: Ventil
- 4: lonisationskammer/Plasmakammer
- 4₁. 4₂,...,4_{N}: Ventil
- 5: Leitung
- 6: Behandlungskammer
- 7: Schleuse
- 8: Objekt
- 9: Leitung
- 10: Entladungsstruktur
- 11: erste Ventilgruppe
- 12: zweite Ventilgruppe
- 13: Leitung
- 14: Pumpe
- 15: Leitung
- 16: Knoten
- 17: Leitung
- 18: Knoten
- 19: Leitung
- 20: Prozessgas
- 21: Gaseinlass
- 22: Gasauslass
- 50: Steuerung
- 52: Kommunikationsverbindung
- 101: Kreislauf
- 102: fluide Verbindung
- 103: fluide Verbindung
- 104: fluide Verbindung
- 105: fluide Verbindung
- 106: fluide Verbindung
- 107: fluide Verbindung
- 108: fluide Verbindung

## Patentansprüche

1. Anordnung (1) zur Behandlung von mindestens einem Objekt (8), **gekennzeichnet durch**:
• eine lonisationskammer/Plasmakammer (4), die über eine Hochspannungsleitung (3) mit der Hochspannungsquelle (2) verbunden ist;
• eine erste Ventilgruppe (11), die mehrere Ventile (3₁, 3₂,...,3_{N}) umfasst und eine zweite Ventilgruppe (12), die mehrere Ventile (4₁, 4₂,...,4_{N}) umfasst, wobei zwischen einem Knoten (16) der erste Ventilgruppe (11) und einem Knoten (18) der zweiten Ventilgruppe (12) eine Pumpe (14) vorgesehen ist;
• eine Behandlungskammer (6), wobei die Behandlungskammer (6) fluide mit der ersten Ventilgruppe (11) über ein Ventil (3₁, 3₂,...,3_{N}) der ersten Ventilgruppe (11) und über ein Ventil (4₁, 4₂,...,4_{N}) der zweiten Ventilgruppe (12) und die lonisationskammer/Plasmakammer (4) fluide mit der ersten Ventilgruppe (11) über ein Ventil (3₁, 3₂,...,3_{N}) der ersten Ventilgruppe (11) und der über ein Ventil (4₁, 4₂,...,4_{N}) zweiten Ventilgruppe (12) verbunden sind; und
• mindestens ein Gaseinlass (21) mit der ersten Ventilgruppe (11) über mindestens ein Ventil (3₁, 3₂,...,3_{N}) der ersten Ventilgruppe (11) und ein Gasauslass (22) über mindestens ein Ventil (4₁, 4₂,...,4_{N}) mit der zweiten Ventilgruppe (12) verbunden sind.

2. Anordnung (1) nach Anspruch 1, wobei die Behandlungskammer (6) über eine Leitung (13) fluide mit einem der Ventile (3₁, 3₂,...,3_{N}) der ersten Ventilgruppe (11) und über eine Leitung (15) fluide mit einem der Ventile (4₁, 4₂,...,4_{N}) der zweiten Ventilgruppe (12) verbunden ist.

3. Anordnung nach einem der vorangehenden Ansprüche 1 bis 2, wobei die lonisationskammer/Plasmakammer (4) über eine Leitung (9) fluide mit einem der Ventile (3₁, 3₂,...,3_{N}) der ersten Ventilgruppe (11) und über eine Leitung (5) fluide mit einem der Ventile (4₁, 4₂,...,4_{N}) der zweiten Ventilgruppe (12) verbunden ist.

4. Anordnung nach einem der vorangehenden Ansprüche 1 bis 2, wobei der Gaseinlass (21) über eine Leitung (17) fluide mit einem der Ventile (3₁, 3₂,...,3_{N}) der ersten Ventilgruppe (11) und der Gasauslass (22) über eine Leitung (19) fluide mit einem der Ventile (4₁, 4₂,...,4_{N}) der zweiten Ventilgruppe (12) verbunden ist.

5. Anordnung (1) nach einem der vorangehenden Ansprüche, wobei eine Steuerung (50) über eine Kommunikationsverbindung (52) mit der Anordnung (1) verbunden ist und, mittels der Steuerung (50), zumindest die Ventile (3₁, 3₂,...,3_{N}) der ersten Ventilgruppe (11), die Ventile (4₁, 4₂,...,4_{N}) der zweiten Ventilgruppe (12), die Hochspannungsquelle (2), die Entladungsstruktur (10) und die Pumpe (14) bedarfs- und zeitgerecht gesteuert und geregelt werden.

6. Verfahren zur Behandlung von mindestens einem Objekt (8), **gekennzeichnet durch** die folgenden Schritte:
• dass das mindestens eine Objekt (8) in eine Behandlungskammer (6) verbracht wird;
• dass mit einer Pumpe (14), die zwischen einem Knoten (16) einer ersten Ventilgruppe (11) mit mehreren Ventilen (3₁, 3₂,...,3_{N}) und einem Knoten (18) einer zweiten Ventilgruppe (12) mit mehreren Ventilen (4₁, 4₂,...,4_{N}) vorgesehen ist, ein Unterdruck in der Behandlungskammer (6) erzeugt wird;
• dass eine lonisationskammer/Plasmakammer (4) mit einem Prozessgas (20) über einen Gaseinlass (21), der mit einem Ventil (3₁, 3₂,...,3_{N}) der ersten Ventilgruppe (11) und einem ein Ventil (4₁, 4₂,...,4_{N}) der zweiten Ventilgruppe (12) verbunden ist, befüllt wird;
• dass eine Entladungsstruktur (10) in der lonisationskammer/Plasmakammer (4) aktiviert wird, so dass eine Ionisation oder Aktivierung des Prozessgases (20) erfolgt;
• dass das aktivierte bzw. ionisierte Prozessgas (20) von der lonisationskammer/Plasmakammer (4) in die Behandlungskammer (6) durch den in der Behandlungskammer (6) herrschenden Unterdruck über ein Ventil (3₁, 3₂,...,3_{N}) der ersten Ventilgruppe (11) und ein Ventil (4₁, 4₂,...,4_{N}) der zweiten Ventilgruppe (12) gesaugt wird;
• dass nach einer Einwirkzeit des aktivierten bzw. ionisierten Prozessgases auf das Objekt (8) die Behandlungskammer (6) abgepumpt und anschließend belüftet wird; und
• dass das mindestens eine behandelte Objekt (8) aus der Behandlungskammer (6) entnommen wird.

7. Verfahren nach Anspruch 6, wobei mittels der Pumpe (14) ein Anreicherungszyklus von aktiviertem bzw. ionisiertem Prozessgas (20) in der lonisationskammer/Plasmakammer (4) durchgeführt wird, dabei die Entladungsstruktur (10) in der lonisationskammer/Plasmakammer (4) aktiviert ist und dabei ein Ventil (3₁, 3₂,...,3_{N}) der ersten Ventilgruppe (11) und ein Ventil (4₁, 4₂,...,4_{N}) der zweiten Ventilgruppe (12) geöffnet sind, so dass die lonisationskammer/Plasmakammer (4) mit der Pumpe (14) durch einen Kreislauf (101) fluide verbunden wird.

8. Verfahren nach einem der vorangehenden Ansprüche 6 bis 7, wobei das mindestens eine Objekt (4) über eine Schleuse (7) in die Behandlungskammer (6) eingebracht wird, wobei ein Ventil (4₁, 4₂,...,4_{N}) der zweiten Ventilgruppe und ein weiteres Ventil (4₁, 4₂,...,4_{N}) der zweiten Ventilgruppe (12) geöffnet sind, so dass die Behandlungskammer (6) eine fluide Verbindung (102) über die zweite Ventilgruppe (12) mit einem Gasauslass (22) ausbildet.

9. Verfahren einem der vorangehenden Ansprüche 6 bis 7, wobei für das Erzeugen des Unterdrucks in der Behandlungskammer (6) die Pumpe (14) aktiviert wird, und dabei ein Ventil (3₁, 3₂,...,3_{N}) der ersten Ventilgruppe (11) und ein Ventil (4₁, 4₂,...,4_{N}) der zweiten Ventilgruppe (12) geöffnet sind, so dass die Behandlungskammer (6) eine fluide Verbindung (103) zu einem Gasauslass (22) ausbildet.

10. Verfahren nach einem der vorangehenden Ansprüche 6 bis 7, wobei zum Befüllen der lonisationskammer/Plasmakammer (4) mit dem Prozessgas (20) ein Ventil (3₁, 3₂,...,3_{N}) der ersten Ventilgruppe (11) und ein Ventil (4₁, 4₂,...,4_{N}) der zweiten Ventilgruppe (12) geöffnet sind, so dass ein Gaseinlass (21) eine fluide Verbindung (104) zu der lonisationskammer/Plasmakammer (4) ausbildet.

11. Verfahren nach einem der vorangehenden Ansprüche 6 bis 7, wobei bei der aktivierten Entladungsstruktur (10) in der lonisationskammer/Plasmakammer (4) eine Ionisation oder Aktivierung des Prozessgases (20) erfolgt, wobei ein Ventil (3₁, 3₂,...,3_{N}) der ersten Ventilgruppe (11) und ein Ventil (4₁, 4₂,...,4_{N}) der zweiten Ventilgruppe (12) geöffnet sind, so dass eine fluide Verbindung (105) von der Behandlungskammer (6) über die Pumpe (14) zum Gasauslass (22) besteht.

12. Verfahren nach einem der vorangehenden Ansprüche 6 bis 7, wobei das aktivierte bzw. ionisierte Prozessgas (20) aus der lonisationskammer/Plasmakammer (4) in die Behandlungskammer (6) gesaugt wird, wobei ein Ventil (3₁, 3₂,...,3_{N}) und ein weiteres Ventil (3₁, 3₂,...,3_{N}) der ersten Ventilgruppe (11) geöffnet sind, so dass eine fluide Verbindung (106) von der lonisationskammer/Plasmakammer (4) zur Behandlungskammer (6) besteht und das Ventil (3₁, 3₂,...,3_{N}) und das weitere Ventil (3₁, 3₂,...,3_{N}) der ersten Ventilgruppe (11) während einer Einwirkzeit geöffnet bleiben.

13. Verfahren nach einem der vorangehenden Ansprüche 6 bis 7, wobei zum Abpumpen der Behandlungskammer (6) ein Ventil (3₁, 3₂,...,3_{N}) der ersten Ventilgruppe (11) und ein Ventil (4₁, 4₂,...,4_{N}) der zweiten Ventilgruppe (12) geöffnet sind, so dass eine fluide Verbindung (107) von der Behandlungskammer (6) über die Pumpe (14) zu einem Gasauslass (22) besteht.

14. Verfahren nach einem der vorangehenden Ansprüche 6 bis 7, wobei zum Belüften der Behandlungskammer (6) ein Ventil (4₁, 4₂,...,4_{N}) und ein weiteres Ventil (4₁, 4₂,...,4_{N}) (43) der zweiten Ventilgruppe (12) geöffnet sind, so dass eine fluide Verbindung (108) von der Behandlungskammer (6) zu einem Gasauslass (22) entsteht.

15. Verfahren nach einem der vorangehenden Ansprüche 6 bis 14, wobei eine Steuerung (50) kommunikativ mit der Anordnung (1) verbunden wird, damit zumindest die Entladungsstruktur (10) der lonisationskammer/Plasmakammer (4), die Ventile (3₁, 3₂,...,3_{N}) der ersten Ventilgruppe (11) und die Ventile (4₁, 4₂,...,4_{N}) der zweiten Ventilgruppe (12) zeitgerecht gesteuert werden.
